# EUROPÄISCHE PATENTSCHRIFT

(11) **EP 3 267 913 B1**
(45) Veröffentlichungstag und Bekanntmachung des Hinweises auf die Patenterteilung: **26.02.2020**
(21) Anmeldenummer: 16709440.8
(22) Anmeldetag: 11.03.2016
(51) Int. Cl.: A61B 17/80, A61B 17/86

(54) **KNOCHENPLATTE MIT EINER KNOCHENSCHRAUBE**
BONE PLATE WITH A BONE SCREW
PLAQUE POUR OS AVEC UNE VIS POUR OS

(30) Priorität: 11.03.2015 DE 102015003087; 29.02.2016 DE 102016002444
(43) Veröffentlichungstag der Anmeldung: 17.01.2018
(73) Patentinhaber: Königsee Implantate GmbH, 07426 Allendorf OT Aschau (DE)
(72) Erfinder: HULLIGER, Urs, 4543 Deitingen (CH); RIESE, Michael, 98708 Gehren (DE); HARZ, Tino, 07426 Königsee-Rottenbach (DE); ORSCHLER, Frank, 99084 Erfurt (DE)
(74) Vertreter: Kruspig, Volkmar
(86) Internationale Anmeldenummer: PCT/EP2016/055237
(87) Internationale Veröffentlichungsnummer: WO 2016/142502

(56) Entgegenhaltungen:
- WO-A1-2011/076205
- DE-B4-102005 042 766
- US-A1- 2013 138 156

## Beschreibung

Die Erfindung betrifft eine Osteosynthesevorrichtung mit einer Knochenplatte sowie mit wenigstens einer Knochenschraube, wobei die Knochenplatte mindestens ein Durchgangsloch mit einem, im Durchgangsloch mindestens teilweise ausgebildeten konischen Innengewinde besitzt, weiterhin die Knochenschraube einen Schraubenschaft sowie einen Schraubenkopf mit einem konischen Gewinde aufweist mit der Möglichkeit der Ausbildung einer variabel winkelstabilen Verbindung zwischen Knochenschraube und Knochenplatte durch Aufnahme des Schraubenkopfes mit konischem Außengewinde im jeweiligen Durchgangsloch der Knochenplatte, wobei das Gewinde im Schraubenkopf einen ersten Abschnitt mit einer ersten Konizität und einen zweiten Abschnitt mit einer zweiten Konizität besitzt, wobei zwischen dem ersten Abschnitt und dem zweiten Abschnitt eine auf die Konizität bezogene Unstetigkeitsstelle vorliegt, gemäß Oberbegriff des Anspruchs 1.

Aus der DE 10 2005 042 766 B4 ist ein Plattenloch einer Knochenplatte für die Osteosynthese vorbekannt. Das Plattenloch weist einen um dessen Umfang verteilten Gewindestern aus radial zur Lochachse zeigenden Gewindeabschnitten mit Gewindezähnen und einer zwischen je zwei benachbarten Gewindezähnen befindlichen Lochausbuchtung sowie einen in Einschraubrichtung sich verjüngenden, konischen Gewindekerndurchmesser auf. Das Plattenloch besitzt eine in Einschraubrichtung oben liegende kalottenförmige Schraubenkopfpfanne zum Erhalt eines umfassenden flächigen Kontakts zwischen einem Schraubenkopf einer Rundkopfschraube und einer Schraubenkopfpfanne derart, dass das Plattenloch zur wahlweisen Aufnahme der Rundkopfschraube oder einer Gewindekopfschraube geeignet ist, um hierdurch im verschraubten Zustand einen variabel bezüglich der Lochlängsachse ausgestalteten Winkel oder eine winkelstabile Verschraubung zu gewährleisten.

Aus der WO 2008/115318 ist ein System zur winkelvariablen Verbindung zwischen einer Knochenplatte und einer oder mehreren Knochenschrauben vorbekannt, wobei die jeweiligen Schraubenköpfe der Knochenschrauben ein Außengewinde aufweisen, dessen Verlauf einem vorgegebenen Radius um einen gedachten Mittelpunkt des Schraubenkopfes entspricht.

In eine ähnliche Lösungsrichtung geht das Knochenplattensystem mit variabler, winkelstabiler Verbindung nach EP 2 559 392 B1, wobei die dort zur Anwendung kommenden Knochenplatten Durchgangslöcher besitzen, die gewindefreie Abschnitte aufweisen und wobei sich die gewindefreien Abschnitte von der Knochenplattenoberseite durchgängig bis zur Knochenplattenunterseite erstrecken.

Bei der Knochenschraube nach DE 10 2006 060 933 A1 weist diese einen eine Längsachse definierenden Schaft und einen als Verdickung ausgeführten Kopf auf, wobei Schaft und Kopf ein Gewinde tragen.

Das Gewinde des Schaftes, das sogenannte Knochengewinde, geht bei dieser Lösung des Standes der Technik unterbrechungsfrei in das Gewinde an dem sich an dem Schaft anschließenden und sich aufweitenden Bereich des Kopfes über. Als Folge liegt ein Teil des Gewinde tragenden Kopfes im Knochengewebe, wodurch eine größere Knochenschraube-Knochen-Kontaktfläche geschaffen werden soll und eine bessere Verteilung und Übertragung von Lasten und Kräften vorliegt.

Bei dem Fixationssystem für Knochen nach DE 198 58 889 B4 ist ein Verbindungsträger in Form einer Knochenplatte mit wenigstens einem Durchgangsloch vorhanden. Darüber hinaus ist wenigstens eine in einem Durchgangsloch eingesetzte Knochenschraube vorhanden, wobei eine gegenseitige Ausrichtung unter verschiedenen Winkeln gegeben ist. Die Mittel zum Festlegen weisen eine durch Eindrehen der Knochenschraube in einem bestimmten Winkel von einem vorgeformten Gewinde unterhalb mindestens einer der Sitzflächen durch Umformung gebildete Gewindeverbindung unterhalb der Sitzflächen von Knochenschraube und Verbindungsträger auf.

Aus der DE 10 2010 038 949 A1 ist eine Osteosynthesevorrichtung mit einer Knochenplatte und wenigstens einer Knochenschraube bekannt, wobei die Knochenplatte wenigstens eine Gewindebohrung aufweist und die Knochenschraube mit einem Schraubenkopf versehen ist, der ein Außengewinde besitzt, wobei weiterhin die Gewindebohrung ein mehrgängiges Innengewinde und die Knochenschraube ein eingängiges Außengewinde aufweist.

Gemäß der dortigen Aufgabenstellung soll die Knochenschraube unter verschiedenen Winkeln in die Gewindebohrung der Knochenplatte einschraubbar sein und es soll die Möglichkeit bestehen, hohe Kräfte auf die Knochenplatte zu übertragen. Gemäß der dortigen Lehre ist vorgesehen, dass die Steigungen des Außengewindes der Knochenschraube und der des Innengewindes der Gewindebohrung gleich groß sind, wodurch nach dem Greifen der Gewinde ein Verspannen erreicht werden soll, welches die Winkelstabilität der Verbindung einleitet.

Um das Einschrauben der vorbekannten Knochenschraube in die Knochenplatte unter verschiedenen Winkeln zu erleichtern, ist vorgesehen, dass die Knochenschraube ein konisches Außengewinde aufweist.

Zur Lösung der Aufgabe nach DE 10 2010 038 949 A1 ist insbesondere vorgesehen, dass das Außengewinde der Knochenschraube wenigstens einen Einschnitt aufweist, der zur Längsachse der Knochenschraube konisch verläuft. Bei einer bevorzugten Ausführungsform sind mehrere Einschnitte, bevorzugt vier Einschnitte, vorgesehen, die gleichmäßig über den Umfang des Außengewindes der Knochenschraube verteilt sind. Die Einschnitte sind kreissegmentförmig ausgestaltet, wobei die Achse des Einschnitts vorzugweise parallel zur Mantellinie des Außengewindes liegt. Die vorerwähnten Einschnitte besitzen die Aufgabe, dass die Gewinde möglichst unmittelbar nach dem Aufsetzen des Außengewindes auf das Innengewinde greifen, ohne dass die Knochenschraube unnötig gedreht werden muss.

In US 2013/138156 A1 wird eine Osteosynthesevorrichtung gemäß dem Oberbegriff des Anspruchs 1 mit einer Knochenplatte offenbart, wobei der Schraubenkopf der Knochenschraube ein konisches Außengewinde aufweist. Das Gewinde im Schraubenkopf weist dabei einen ersten Abschnitt mit einer ersten Konizität und einen zweiten Abschnitt mit einer zweiten Konizität auf, wobei zwischen den beiden Abschnitten eine auf die Konizität bezogene Unstetigkeitsstelle vorliegt.

Aus dem Vorgenannten ist es Aufgabe der Erfindung, eine weiterentwickelte Osteosynthesevorrichtung mit einer Knochenplatte sowie mit mindestens einer Knochenschraube anzugeben, wobei die Knochenplatte mindestens ein Durchgangsloch mit einem, im Durchgangsloch mindestens teilweise ausgebildeten konischen Innengewinde besitzt. Die weiterentwickelte Osteosynthesevorrichtung soll ein günstiges Eindrehmoment und eine hohe Verriegelungsstabilität besitzen. Insbesondere ist zu gewährleisten, dass auch dann, wenn die Knochenschraube senkrecht zur Längsachse der Knochenplatte in das Durchgangsloch eingebracht wird, eine sehr stabile Verriegelung gegeben ist, die mindestens derjenigen entspricht, welche bei Osteosynthesevorrichtungen mit nicht winkelvariabler Ausgestaltung der betreffenden Schraubverbindungen vorliegt.

Die Lösung der Aufgabe der Erfindung erfolgt durch die Merkmalskombination gemäß der Lehre des Patentanspruchs 1 und der dort beschriebenen Osteosynthesevorrichtung, bestehend aus Knochenplatte und wenigstens einer Knochenschraube, wobei die Unteransprüche mindestens zweckmäßige Ausgestaltungen und Weiterbildungen darstellen.

Es wird demnach von einer Osteosynthesevorrichtung mit einer Knochenplatte sowie mit wenigstens einer Knochenschraube ausgegangen, wobei die Knochenplatte mindestens ein Durchgangsloch mit einem im Durchgangsloch mindestens teilweise ausgebildeten konischen Außengewinde besitzt. Die Definition Knochenplatte und Durchgangsloch mit mindestens teilweise ausgebildetem konischen Gewinde soll nicht einschränkend verstanden werden, sondern es können selbstverständlich völlig unterschiedliche, anatomisch angepasste Knochenplatten mit einer Vielzahl von Durchgangslöchern vorliegen, wobei mindestens eines der Durchgangslöcher das erwähnte konische Innengewinde besitzt.

Maßgeblich ist, dass die Knochenschraube einen Schraubenschaft sowie einen Schraubenkopf mit einem konischen Außengewinde aufweist mit der Möglichkeit der Ausbildung einer variabel winkelstabilen Verbindung zwischen Knochenschraube und Knochenplatte durch Aufnahme des Schraubenkopfes mit konischem Außengewinde in das jeweilige Durchgangsloch der Knochenplatte.

Das Außengewinde ist im Schraubenkopf besonders ausgestaltet dahingehend, dass ein erster Abschnitt A mit einer ersten Konizität und ein zweiter Abschnitt B mit einer zweiten Konizität versehen ist, wobei zwischen dem ersten Abschnitt A und dem zweiten Abschnitt B eine auf die Konizität bezogene Unstetigkeitsstelle vorliegt.

Unter Unstetigkeitsstelle wird hierbei ein Übergang von der ersten Konizität zur zweiten Konizität verstanden, der abrupt verläuft. Die Unstetigkeitsstelle kann dabei einen ersten Unstetigkeitspunkt mit einem folgenden stetigen Verlauf und einen daran anschließenden zweiten Unstetigkeitspunkt besitzen, wobei zwischen erstem und zweitem Unstetigkeitspunkt keine Konizität des Gewindes vorliegt.

Es liegt weiterhin im Sinne der Erfindung, dass dem ersten und zweiten Abschnitt ein dritter Abschnitt mit wiederum geänderter Konizität folgt, ohne den Grundgedanken der Erfindung zu verlassen.

Erfindungsgemäß ist der erste Abschnitt A zum Ende des Schraubenkopfes und der zweite Abschnitt B zum Schraubenschaft gerichtet. Unter Ende des Schraubenkopfes wird die Schraubenkopfseite verstanden, die der Aufnahme bzw. dem An- oder Einsetzen eines Werkzeugs zum Betätigen der Schraube dient.

In besonders erfindungsgemäßer Weise ist das Gewinde im Schraubenkopf als doppelkonisches bzw. mehrfach konisches Gewinde ausgebildet, wobei der Konus im ersten Abschnitt A kleiner als der Konus im zweiten Abschnitt B realisiert ist.

Der kleinere Konus bzw. Konuswinkel entspricht im Wesentlichen der Konizität des Gewindes im Durchgangsloch.

In bevorzugter Weiterbildung der Erfindung liegt der Konuswinkel der ersten Konizität bei im Wesentlichen 10° bis 20°, bevorzugt 14° bis 16°, und der Konuswinkel der zweiten Konizität bei im Wesentlichen 23° bis 30°, bevorzugt 24° bis 26°.

Durch den größeren Konuswinkel und damit steileren Konus erfolgt bei einem, bevorzugt auf den von der Senkrechten der Knochenplatte abweichenden Neigungswinkel von bis zu 15° ein selbständiges Einziehen der Knochenschraube in das Durchgangslochgewinde.

Die Osteosynthesevorrichtung umfasst erfindungsgemäß speziell ausgebildete Innengewinde in dem oder den Durchgangslöchern der Knochenplatte derart, dass mehrere zur Knochenplattenoberseite bzw. zur Einschraubseite gerichtete, umfangsseitig verteilt angeordnete Freistellungen in Form von gewindefreien Rücksprüngen ausgebildet sind, welche sich auch nur über einen Teil der Tiefe des jeweiligen Durchgangslochs erstrecken können. Grundsätzlich soll jedoch die Tendenz erhalten bleiben, dass die Gewindeunterbrechung zur Plattenunterseite abnimmt.

Dies bedeutet, dass das Innengewinde im jeweiligen Durchgangsloch der Knochenplatte im Bereich zur Knochenplattenunterseite im Wesentlichen unterbrechungsfrei ausgebildet ist.

Die Erfindung soll nachstehend anhand eines Ausführungsbeispiels sowie unter Zuhilfenahme von Figuren näher erläutert werden.

Hierbei zeigen:
- Fig. 1: eine Schnittdarstellung der erfindungsgemäßen Osteosynthesevorrichtung mit Knochenplatte sowie einer Knochenschraube, wobei die Knochenplatte ein Durchgangsloch mit konischem Innengewinde aufweist;
- Fig. 2: eine Detaildarstellung auf der Basis der Fig. 1 mit den Abschnitten A und B, wobei der Abschnitt A als Haltebereich und der Abschnitt B als Einziehbereich definiert ist, und zwar im Sinne eines selbständigen Einziehens der entsprechenden Knochenschraube in das Gewinde im jeweiligen Durchgangsloch;
- Fig. 3: eine Schnittdarstellung mit erkennbarem Verblocken des Schraubengewindes in der Neigungsebene über ausgeprägte Ecken in den Regionen X und Y;
- Fig. 4: eine Schnittdarstellung längs der Linien A-A nach Fig. 3 mit dort erkennbarer Verblockung über den gesamten Konusbereich in den Regionen Z, wobei die Schnittdarstellung nach Fig. 4 in einem Winkel von 90° zu der Darstellung nach Fig. 3 gewählt ist;
- Fig. 5: eine Schnittdarstellung der Verblockung mit verbessertem Haltemoment;
- Fig. 6: eine Erläuterungsskizze bezüglich der Verdrängungsquerschnitte beim Ausbilden einer festen Verbindung zwischen Knochenschraube und Knochenplatte;
- Fig. 7: eine Schnittdarstellung durch eine Knochenplatte mit erfindungsgemäßem Durchgangsloch, welches ein konisches Innengewinde aufweist und wobei eine Freistellung in Form eines gewindefreien Rücksprungs vorgesehen ist,
- Fig. 8: eine Darstellung ähnlich derjenigen nach Fig. 7, jedoch teilweggebrochen und teils perspektivisch, und
- Fig. 9a bis f: verschiedene Möglichkeiten der Ausgestaltung der Freistellungen in Form von gewindefreien Rücksprüngen.

Die in den Figuren beschriebene Osteosynthesevorrichtung geht von einer Knochenplatte 1 sowie wenigstens einer Knochenschraube 2 aus, wobei die Knochenplatte 1 mindestens ein Durchgangsloch 3 mit einem im Durchgangsloch mindestens teilweise ausgebildeten konischen Innengewinde 4 besitzt.

Die Knochenschraube 2 weist einen Schraubenschaft 5 sowie einen Schraubenkopf 6 auf.

Der Schraubenkopf 6 besitzt ein spezielles konisches Gewinde mit der Möglichkeit der Ausbildung einer variabel winkelstabilen Verbindung zwischen Knochenschraube 2 und Knochenplatte 1 durch Aufnahme des Schraubenkopfes 6 mit dem speziellen konischen Außengewinde im jeweiligen Durchgangsloch 3 der Knochenplatte 1.

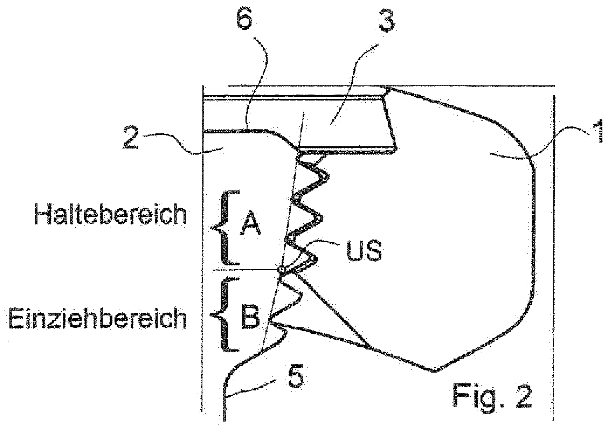

Das Gewinde im Schraubenkopf 5 besitzt einen ersten Abschnitt A (siehe Fig. 2), dort als Haltebereich bezeichnet, mit einer ersten Konizität und einen zweiten Abschnitt B, dort als Einziehbereich bezeichnet, mit einer zweiten Konizität, wobei zwischen dem ersten Abschnitt A und dem zweiten Abschnitt B eine auf die Konizität bezogene Unstetigkeitsstelle US vorliegt.

Der erste Abschnitt A ist zum Ende des Schraubenkopfes und der zweite Abschnitt B zum Schraubenschaft 5 gerichtet.

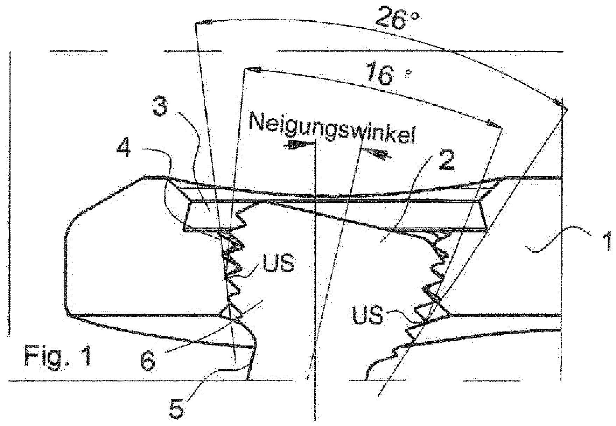

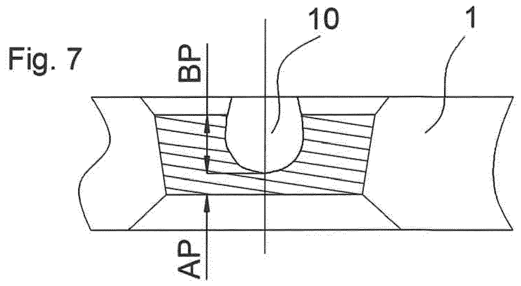

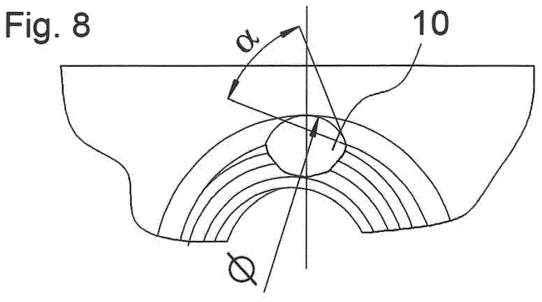

Bei einer bevorzugten Ausführungsform der Konuswinkel, wie in der Fig. 1 dargestellt, besitzt das konische Gewinde in der Knochenplatte 1 eine Konizität von 16°, wobei das Gewinde in der Knochenplatte 1 zweigängig realisiert ist. Bei einer entsprechenden Ausgestaltung weist darüber hinaus das konische Gewinde in der Knochenplatte 1 bzw. im Durchgangsloch 3 der Knochenplatte 1 fünf umfangsseitig verteilte Freistellungen z.B. in V-Form am Gewindeumfang auf, wie dies auch anhand der Fig. 7 und 8 dargestellt ist.

Der Schraubenkopf 6 besitzt gemäß der Darstellung nach den Figuren, insbesondere der Fig. 1, ein doppelkonisches Kopfgewinde, und zwar von 16° einerseits sowie 22° bis 26° andererseits.

Der kleinere Konus von 16° am Kopfgewinde entspricht dem Konuswinkel von 16° im Durchgangsloch 3 der Knochenplatte 1.

Das Schraubengewindeprofil entspricht dem Profil des Gewindes in der Knochenplatte 1. Der in der Fig. 1 dargestellte Neigungswinkel kann im Bereich von 0° bis 15° gewählt werden. Im Falle eines Neigungswinkels von 0° ist die Knochenschraube senkrecht zur Längsachse der Knochenplatte 1 in das gewindebehaftete Durchgangsloch 3 eingebracht.

Durch den zweiten, steileren Konus von 22° bis 26° am Schraubenkopf 6, in der Fig. 2 mit B bezeichnet, ist bei einem großen Neigungswinkel der Knochenschraube 2 zur Knochenplatte 1 ein selbständiges Einziehen der Knochenschraube 2 in das Gewinde im jeweiligen Durchgangsloch 3 der Knochenplatte 1 sichergestellt.

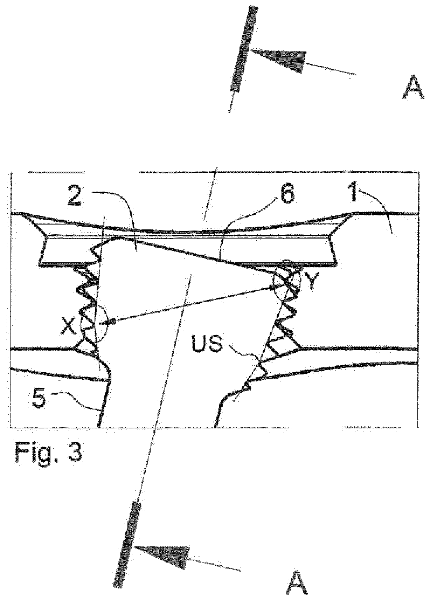

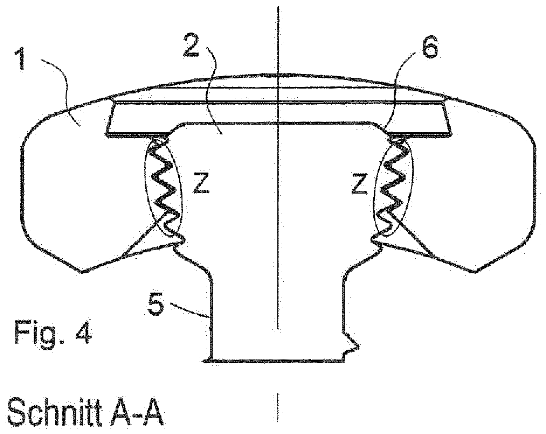

Durch das selbständige Einziehen der Knochenschraube in das Gewinde im Durchgangsloch 3 der Knochenplatte 1 wird einerseits ein Formschluss des Schraubengewindes mit dem Plattengewinde in der Neigungsebene der Schraube gemäß der Darstellung nach Fig. 3 und andererseits ein Formschluss des Schraubengewindes mit dem Plattengewinde gemäß Schnitt A-A nach Fig. 4 (90° versetzt) erreicht.

In den Bereichen dazwischen werden beide Gewinde durch eine teilweise Deformation / Umformung der Gewindespitzen verbunden.

Bei Verwendung einer gehärteten Knochenschraube und einer diesbezüglich nicht gehärteten Knochenplatte wird nur das Gewinde im Durchgangsloch der Knochenplatte umgeformt, wobei die Knochenschraube hingegen unbeschädigt bleibt. Eine stabile Verriegelung ist jedoch auch mit Materialien gleicher Härte bezüglich Knochenplatte und Knochenschraube möglich.

Das Verblocken des Schraubengewindes geschieht in der Neigungsebene über die ausgeprägten Ecken nach Fig. 3, Regionen X und Y, und 90° dazu über den gesamten Konusbereich, siehe Fig. 4, Regionen Z. Durch eine optimierte Ausgestaltung der konischen Bereiche mit Unstetigkeitsstelle wird eine optimale Haltekraft bei minimalem Eindrehmoment der Gewindeverbindung erreicht.

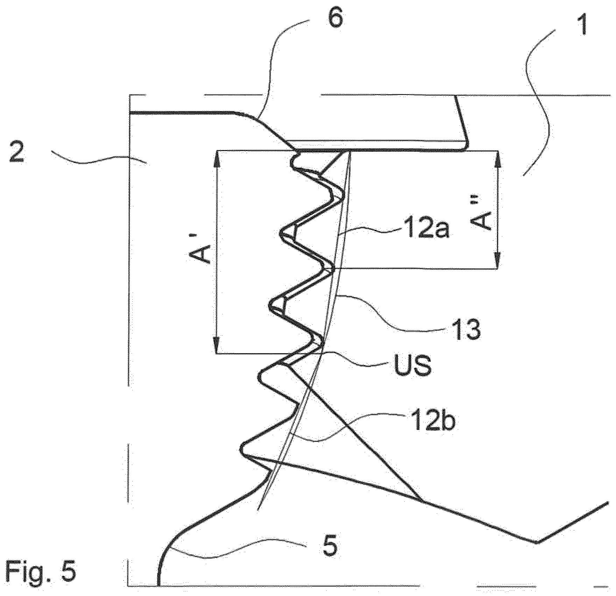

Im Gegensatz zu einer Ausgestaltung mit Radius des Schraubenkopfgewindes wird ein verbessertes Haltemoment durch die Unstetigkeitsstelle im Sinne einer "eckigen" Ausführung erreicht. Der konische Bereich, welcher bei vollständig eingeschraubter Schraube zum Tragen kommt, ist gegenüber einer Variante mit Radius nach dem Stand der Technik deutlich verbessert, wie dies die Fig. 5 nebst dortigen kurzen Erläuterungen symbolisiert. Es ist mithin der Bereich A' deutlich größer als der Bereich A".

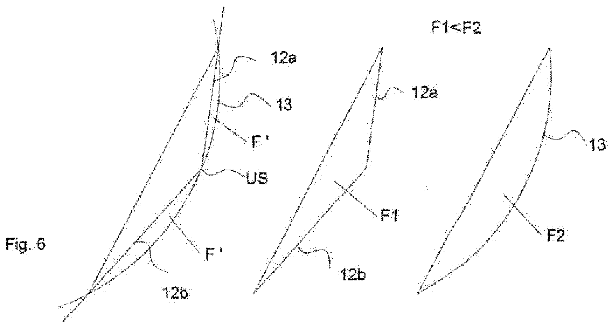

Bezogen auf die Figuren 5 und 6 bezeichnet die Bezugsziffer 12A die einhüllende der Gewindespitzen der doppelkonischen Schraube im Gewindebereich A, d. h. dem Haltebereich, das Bezugszeichen 12B die Einhüllende der Gewindespitzen der doppelkonischen Schraube im Gewindebereich B, d. h. im Einziehbereich, und das Bezugszeichen 13 die Einhüllende der Gewindespitzen der Schraube mit Radius des Schraubenkopfgewindes.

Ein weiterer Vorteil der erfindungsgemäßen Ausführung besteht darin, dass das Einschraubmoment der Knochenschraube in die Knochenplatte durch einen verkleinerten Verdrängungsquerschnitt reduziert ist, was aus den Darstellungen nach Fig. 6 ersichtlich ist. Die Fläche des Dreieckes F1 gemäß Figur 6, welche den Verdrängungsquerschnitt der doppelkonischen Schraube repräsentiert, ist um die Fläche F' kleiner als die Fläche des vergleichbaren Dreiecks F2, welche den Verdrängungsquerschnitt der Schraube mit Radius des Schraubenkopfgewindes repräsentiert. Vorstehendes führt zu einer geringeren Verdrängungsarbeit mit der Folge eines geringeren Einschraubwiderstandes bei der Erzeugung einer stabilen Verriegelung gemäß der erfindungsgemäßen Lösung.

Mit Hilfe der Fig. 7 und 8 soll der Aufbau der Freistellungen in Form von gewindefreien Rücksprüngen in dem betreffenden Durchgangsloch der Knochenplatte erläutert werden.

Die Freistellungen 10, welche umfangsseitig in dem entsprechenden Durchgangsloch verteilt angeordnet sind, stellen sicher, dass das Gewinde der Knochenschraube bei Schrägstellung sich am Plattengewinde festsetzen kann und die Knochenschraube beim Festziehen in das Plattengewinde eingezogen wird.

Durch die bezogen auf die Tiefe des Durchgangslochs bzw. die Plattendicke nicht durchgehende Ausbildung der Freistellungen 10 oder die abnehmende Ausdehnung der Freistellungen in Richtung Plattenunterseite entstehen im Plattengewinde zwei Bereiche. Der Bereich BP ermöglicht das Einziehen der Knochenschraube in das Plattengewinde. Im Bereich AP wird eine verbesserte Verblockung der Knochenschraube im Plattengewinde erreicht.

Durch die nicht durchgehende Gestaltung oder zunehmende Verkleinerung der Freistellungen wird zudem das maximale Durchdrehmoment der Schraube durch die Platte erhöht, da der Bereich AP ein zunehmend intaktes, komplettes Plattengewinde aufweist. Im Gegensatz zu Freistellungen des Standes der Technik mit Übergangswinkeln vom Ausschnitt zum Gewinde ermöglicht der flachere Keil der erfindungsgemäßen Lösung ein leichteres Eindringen des Schraubengewindes in das Plattengewinde. Hieraus ergibt sich eine verbesserte Umformbarkeit der Gewinde und ein Verhindern ansonsten störender Spanbildung. Weiterhin führt die vorstehend erläuterte Ausgestaltung zu einer Reduzierung des Eindrehmoments der Schraube.

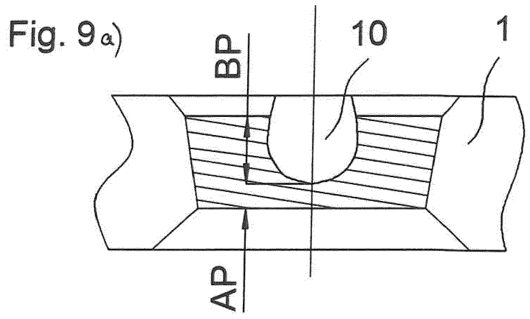

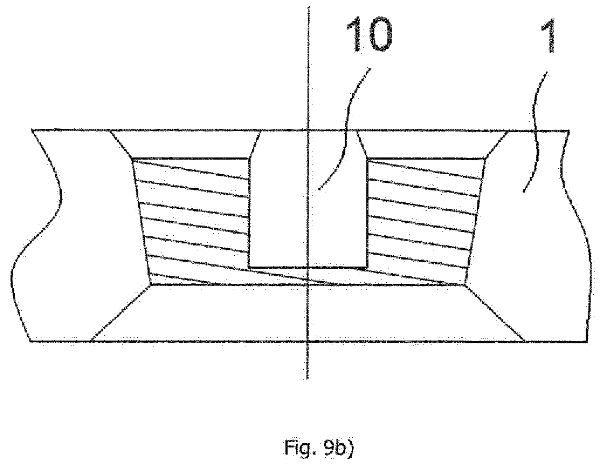

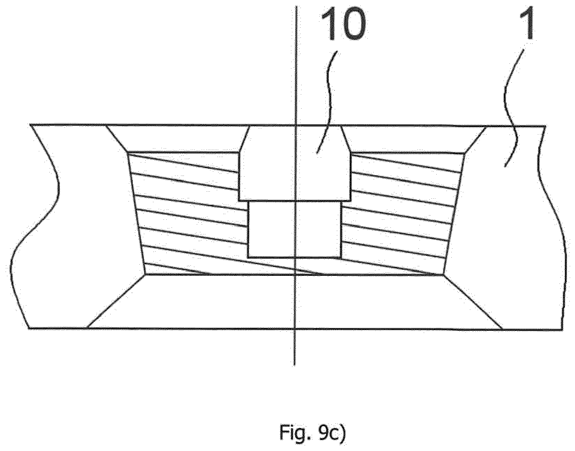

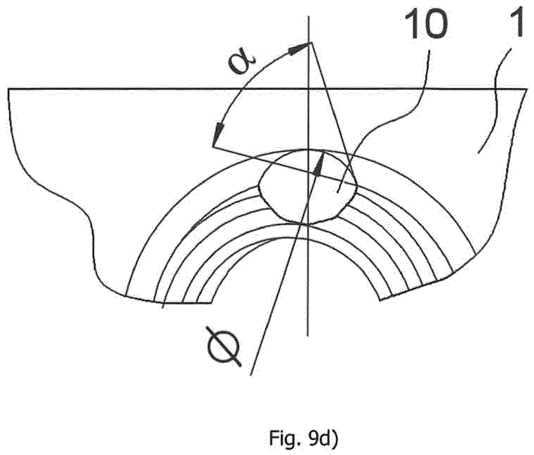

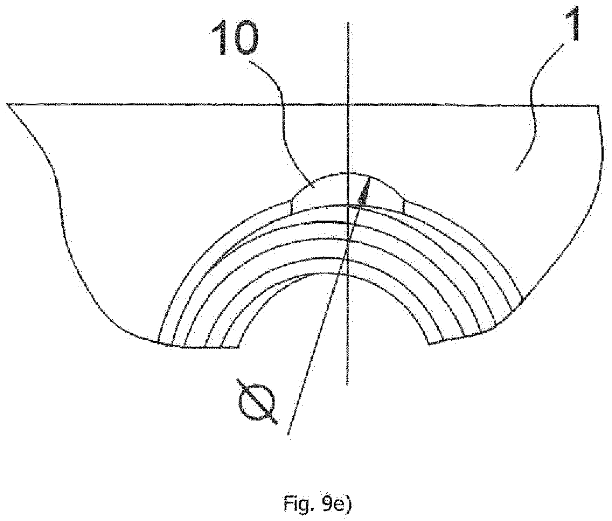

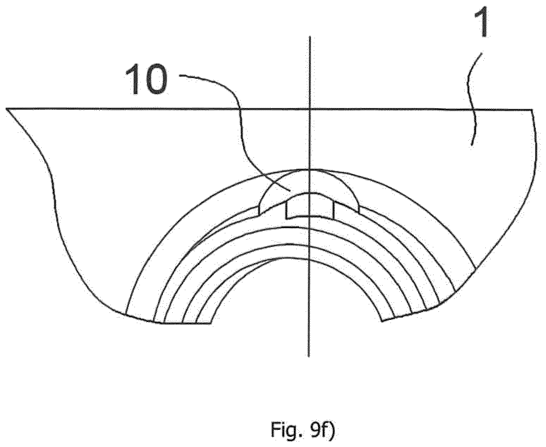

Die Fig. 9 zeigt ausgehend von den Fig. 7 und 8 weitere mögliche Ausgestaltungen und geometrische Konfigurationen der Freistellungen 10. Die Freistellungen 10 sind so zu gestalten, dass der in der Fig. 9 gezeigte Übergangswinkel α < 40° ist.

Der Flankenwinkel der Gewinde in der Platte und der jeweiligen Schraube beträgt üblicherweise 60°. Von dieser Standard-Konfiguration können erfindungsgemäß Abweichungen erfolgen, beispielsweise im Bereich von 50° bis 70°. Die Gewindeflanken können darüber hinaus beim Plattengewinde einen anderen Flankenwinkel aufweisen, als es beim Schraubengewinde der Fall ist. Beispielsweise kann bei einer Schraube größerer Härte ein kleinerer Winkel im Vergleich zum Flankenwinkel der Platte gewählt werden, um eine bessere Verblockung zu gewährleisten. Beispielsweise kann bei einer relativ weichen Knochenplatte und einer Schraube größer Härte eine Anpassung des Flankenwinkels der Platte von im Wesentlichen 65° und der Schraube von im Wesentlichen 55° vorgenommen werden, um das Eindrehverhalten und die Verblockung zu optimieren.

Der Flankenwinkel der Gewinde in der Platte und der jeweiligen Schraube beträgt üblicherweise 60°. Von dieser Standard-Konfiguration können erfindungsgemäß Abweichungen erfolgen, beispielsweise im Bereich von 50° bis 70°. Die Gewindeflanken können darüber hinaus beim Plattengewinde einen anderen Flankenwinkel aufweisen, als es beim Schraubengewinde der Fall ist. Beispielsweise kann bei einer Schraube größerer Härte ein kleinerer Winkel im Vergleich zum Flankenwinkel der Platte gewählt werden, um eine bessere Verblockung zu gewährleisten. Beispielsweise kann bei einer relativ weichen Knochenplatte und einer Schraube größer Härte eine Anpassung des Flankenwinkels der Platte von im Wesentlichen 65° und der Schraube von im Wesentlichen 55° vorgenommen werden, um das Eindrehverhalten und die Verblockung zu optimieren.

## Patentansprüche

1. Osteosynthesevorrichtung mit einer Knochenplatte (1) sowie mit wenigstens einer Knochenschraube (2), wobei die Knochenplatte (1) mindestens ein Durchgangsloch (3) mit einem, im Durchgangsloch (3) mindestens teilweise ausgebildeten konischen Innengewinde (4) besitzt, weiterhin die Knochenschraube (2) einen Schraubenschaft (5) sowie einen Schraubenkopf (6) mit einem konischen Gewinde aufweist mit der Möglichkeit der Ausbildung einer variabel winkelstabilen Verbindung zwischen Knochenschraube (2) und Knochenplatte (1) durch Aufnahme des Schraubenkopfes (6) mit konischem Außengewinde im jeweiligen Durchgangsloch (3) der Knochenplatte (1), wobei
das Gewinde im Schraubenkopf (6) einen ersten Abschnitt (A) mit einer ersten Konizität und einen zweiten Abschnitt (B) mit einer zweiten Konizität besitzt, wobei zwischen dem ersten Abschnitt (A) und dem zweiten Abschnitt (B) eine auf die Konizität bezogene Unstetigkeitsstelle (US) vorliegt,
**dadurch gekennzeichnet, dass**
das Innengewinde im jeweiligen Durchgangsloch (3) der Knochenplatte (1) mehrere, zur Knochenplattenoberseite bzw. zur Einschraubseite gerichtete, umfangsseitig verteilt angeordnete Freistellungen (10) in Form von gewindefreien Rücksprüngen aufweist, wobei das Innengewinde im jeweiligen Durchgangsloch (3) der Knochenplatte (1) im Bereich der Knochenplattenunterseite unterbrechungsfrei ausgebildet ist.

2. Osteosynthesevorrichtung nach Anspruch 1,
**dadurch gekennzeichnet, dass**
der erste Abschnitt (A) zum Ende des Schraubenkopfes (6) und der zweite Abschnitt (B) zum Schraubenschaft (5) gerichtet ist.

3. Osteosynthesevorrichtung nach Anspruch 1 oder 2,
**dadurch gekennzeichnet, dass**
das Gewinde im Schraubenkopf (6) als doppelkonisches Gewinde ausgebildet ist, wobei der Konus im ersten Abschnitt (A) kleiner als der Konus im zweiten Abschnitt (B) realisiert ist.

4. Osteosynthesevorrichtung nach Anspruch 3,
**dadurch gekennzeichnet, dass**
der kleinere Konus bzw. Konuswinkel im Wesentlichen der Konizität des Gewindes im Durchgangsloch (3) entspricht.

5. Osteosynthesevorrichtung nach einem der vorangegangenen Ansprüche,
**dadurch gekennzeichnet, dass**
der Konuswinkel der ersten Konizität bei im Wesentlichen 10° bis 20°, bevorzugt 14° bis 16° und der Konuswinkel der zweiten Konizität bei im Wesentlichen 23° bis 30°, bevorzugt 24° bis 26° liegt.

## Claims

1. Osteosynthesis device with a bone plate (1) and with at least one bone screw (2), wherein the bone plate (1) has at least one through-hole (3) with a conical inner thread (4) formed at least in part in the through-hole (3), the bone screw (2) moreover has a screw shank (5) and also a screw head (6) with a conical thread, with the possibility of forming a connection at a variable and stable angle between the bone screw (2) and the bone plate (1) by receiving the screw head (6) with the conical outer thread in the respective through-hole (3) of the bone plate (1), wherein the thread in the screw head (6) has a first portion (A) with a first conicity and a second portion (B) with a second conicity, wherein between the first portion (A) and the second portion (B) there is a point of discontinuity (US) with regard to the conicity, **characterized in that** the inner thread in the respective through-hole (3) of the bone plate (1) has a plurality of clearances (10) in the form of thread-free recesses which are distributed around the circumference and which are directed towards the bone plate upper side or towards the screw-in side, wherein the inner thread in the respective through-hole (3) of the bone plate (1) is designed to be free of interruptions in the region of the bone plate lower side.

2. Osteosynthesis device according to claim 1, **characterized in that** the first portion (A) is directed towards the end of the screw head (6) and the second portion (B) is directed towards the screw shank (5).

3. Osteosynthesis device according to claim 1 or 2, **characterized in that** the thread in the screw head (6) is designed as a double-conical thread, wherein the cone in the first portion (A) is smaller than the cone in the second portion (B).

4. Osteosynthesis device according to claim 3, **characterized in that** the smaller cone or cone angle corresponds substantially to the conicity of the thread in the through-hole (3).

5. Osteosynthesis device according to any one of the preceding claims, **characterized in that** the cone angle of the first conicity is substantially 10° to 20°, preferentially 14° to 16°, and the cone angle of the second conicity is substantially 23° to 30°, preferentially 24° to 26°.

## Revendications

1. Dispositif d'ostéosynthèse comprenant une plaque à os (1) et au moins une vis à os (2),
dans lequel
la plaque à os (1) présente au moins un trou traversant (3) avec un taraudage conique (1) au moins partiellement formé dans le trou traversant (3),
en outre, la vis à os (2) présente une tige de vis (5) et une tête de vis (6) ayant un filetage conique, avec la possibilité de former une liaison angulairement stable et variable entre la vis à os (2) et la plaque à os (1) en recevant la tête de vis (6) à filetage conique dans le trou traversant respectif (3) de la plaque à os (1),
le filetage dans la tête de vis (6) présente une première portion (A) avec une première conicité et une deuxième portion (B) avec une deuxième conicité,
entre la première portion (A) et la deuxième portion (B), il y a un point de discontinuité (US) relative à la conicité,
**caractérisé en ce que**
le filetage dans le trou traversant respectif (3) de la plaque à os (1) présente plusieurs dégagements (10) répartis sur la circonférence sous la forme de retraits dépourvus de taraudage et dirigés vers la face supérieure de la plaque à os ou vers le côté de vissage,
le taraudage dans le trou traversant respectif (3) de la plaque à os (1) est réalisé sans interruption dans la zone de la face inférieure de la plaque à os (1).

2. Dispositif d'ostéosynthèse selon la revendication 1,
**caractérisé en ce que**
la première portion (A) est dirigée vers l'extrémité de la tête de vis (6) et la deuxième portion (B) est dirigée vers la tige de vis (5).

3. Dispositif d'ostéosynthèse selon la revendication 1 ou 2,
**caractérisé en ce que**
le filetage dans la tête de vis (6) est réalisé comme un filetage double conique, le cône dans la première portion (A) étant plus petit que le cône dans la deuxième portion (B).

4. Dispositif d'ostéosynthèse selon la revendication 3,
**caractérisé en ce que**
le petit cône ou angle de cône correspond sensiblement à la conicité du taraudage dans le trou traversant (3).

5. Dispositif d'ostéosynthèse selon l'une des revendications précédentes,
**caractérisé en ce que**
l'angle de cône de la première conicité est sensiblement de 10° à 20°, de préférence de 14° à 16°, et l'angle de cône de la deuxième conicité est sensiblement de 23° à 30°, de préférence de 24° à 26°.
